# EUROPEAN PATENT APPLICATION

(11) **EP 2 103 320 A1**
(43) Date of publication of application: **23.09.2009**
(21) Application number: 07831644.5
(22) Date of filing: 12.11.2007
(51) Int. Cl.: A61M 5/315

(54) **PISTON FOR SYRINGE**

(30) Priority: 27.12.2006 JP 2006353082
(71) Applicant: Daikyo Seiko, LTD., Tokyo 131-0031 (JP)
(72) Inventor: SUDO, Morihiro, Sumida-ku Tokyo 131-0031 (JP); KOSIDAKA, Tsuyoshi, Sumida-ku Tokyo 131-0031 (JP)
(74) Representative: Piésold, Alexander James
(86) International application number: PCT/JP2007/071914
(87) International publication number: WO 2008/078467

(57) **Abstract**

For a syringe piston that is made of a rubber in a generally cylindrical shape and that is slidably mounted in a syringe barrel,
the syringe piston in a generally cylindrical shape is **characterized by** comprising a circular depression at an almost intermediate part thereof,
wherein R1 is smaller than R2 in the case in which sliding surfaces are disposed on the both sides of the circular depression and R1 is a diameter of the sliding surface on the leading end side of the piston and R2 is a diameter of the sliding surface on the rear end side of the piston,
L1 is equivalent to or larger than L2 in the case in which L1 is a length of the sliding surface on the leading end side of the piston and L2 is a length of the sliding surface on the rear end side of the piston, and
r is equivalent to or larger than 0.7 mm in the case in which a radius of curvature of a corner portion on the leading end side of the piston is r.

## Description

### TECHNICAL FIELD

The present invention relates to a syringe piston for a medical drug and a medical treatment. More specifically, the present invention relates to a syringe piston that has a high sealing performance to a medicinal solution filled into a syringe in storage and in use and that has an excellent sliding performance in use.

### BACKGROUND ART

It is necessary that a syringe piston for a medical drug and a medical treatment does not interact with a medicinal solution filled into a syringe and that a syringe piston for a medical drug and a medical treatment is provided with conflicting characteristics (performances) of a sealing performance and a sliding performance.

In recent years in particular, for an increasing number of pistons of a prefilled syringe (container and syringe) that is filled with a medicinal solution in advance, the above characteristics are required at a level higher than that of a normal syringe piston. In addition, it is also required that a quality does not vary, and that an excellent sealing performance (safety) to a medicinal solution and an excellent sliding property at a level in which a healthcare professional can easily use the syringe are also ensured in a long expiration date for a safe use.

To satisfy the above requests, a piston having surfaces (a medicinal solution contact surface and a sliding surface) that are laminated by a fluorine resin or the like has been known for instance (see Patent document 1).

For a conventional laminate piston, it is general that a diameter on the leading end side of the piston is larger than that on the rear end side of the piston, and that a length of a sliding surface on the leading end side of the piston is larger than that on the rear end side of the piston (see Patent document 1 for instance).

This is caused by that the functions of the sliding surfaces are different from each other in the case in which a piston is inserted into a syringe barrel. In other words, it is important that a sliding surface on the leading end side of the piston has a function for holding a sealing performance in such a manner that a medicinal solution is prevented from leaking to the rear end side of the piston. Consequently, it is preferable that a diameter of a sliding surface on the leading end side of the piston is larger than that on the rear end side of the piston, and that a length of a sliding surface on the leading end side of the piston is larger than that on the rear end side of the piston.

On the other hand, it is important that a sliding surface on the rear end side of the piston has a function for controlling a posture of the piston. In consideration of that a sliding resistance of the piston is affected to a large degree by an area of contact and a contact pressure between the piston and an inner wall of the syringe barrel, it is preferable that a diameter of a sliding surface on the rear end side of the piston is smaller than that on the leading end side of the piston, and that a length of a sliding surface on the rear end side of the piston is smaller than that on the leading end side of the piston.

As a matter of course, for the above requirements, in the case in which a non laminate piston (a piston that is not laminated) that can utilizes a sealing performance of a rubber elasticity to the maximum extent possible is used or it is hard to leak a medicinal solution due to a property of the medicinal solution (such as a high viscosity), it is known that it is permissible that a diameter of a sliding surface on the leading end side of the piston is equivalent to that on the rear end side of the piston, and that a length of a sliding surface on the leading end side of the piston is equivalent to that on the rear end side of the piston.
Patent document 1: Japanese Patent Application Laid-Open Publication No. 2003-190285

### DISCLOSURE OF THE INVENTION

### PROBLEMS TO BE SOLVED BY THE INVENTION

However, due to a progress of science and technology in recent years, a large increase in speed of a medicinal solution filling line has progressed in a pharmaceutical manufacturer, and the problem of ununiformity of a filling posture of a piston involved in a high speed filling has occurred.

In the case in which a conventional piston having the above described mode is used and the piston is inserted into a syringe barrel at an angle, a slight gap is generated between the piston and the inner wall of the syringe barrel, thereby causing.a medicinal solution to leak disadvantageously in some cases.

In the case in which a healthcare professional carries out an injection operation, the piston is engage with a plunger rod for a use in general. However, in the case in which the engagement is inappropriate or the wrong operation of pressing the plunger rod is carried out whereby a force in a direction other than the axial direction of the piston is applied to the piston, an inserting posture of the piston becomes turbulent and a medicinal solution leaks from a gap between the piston and the inner wall of the syringe barrel in some cases.

The present invention was made in consideration of such conditions, and an object of the present invention is to provide a syringe piston having a high sealing performance caused by a stable inserting position of the piston and having a sliding resistance that does not increase even in the case in which a force for disturbing an inserting posture of the piston is applied to the piston in a filling or use of the piston.

### MEANS FOR SOLVING THE PROBLEMS

For a syringe piston that is made of a rubber in a generally cylindrical shape and that is slidably mounted in a syringe barrel,
the syringe piston in a generally cylindrical shape in accordance with the present invention is characterized by comprising a circular depression at an almost intermediate part thereof,
wherein R1 is smaller than R2 in the case in which sliding surfaces are disposed on the both sides of the circular depression and R1 is a diameter of the sliding surface on the leading end side of the piston and R2 is a diameter of the sliding surface on the rear end side of the piston,
L1 is equivalent to or larger than L2 in the case in which L1 is a length of the sliding surface on the leading end side of the piston and L2 is a length of the sliding surface on the rear end side of the piston, and
r is equivalent to or larger than 0.7 mm in the case in which a radius of curvature of a corner portion on the leading end side of the piston is r.

The syringe piston in accordance with the present invention is **characterized in that** a flat part E is preferably formed on the sliding surface on the rear end side of the piston.

For the piston having the above configuration, a corner portion on the leading end side of the piston is a gentle curved surface. Consequently, a sliding resistance of the sliding surface on the leading end side of the piston is reduced, and a sealing performance holding ability of the corner portion can be improved even in the case in which the piston posture slants. Moreover, since R1 is smaller than R2, a stability of an inserting posture of the piston can be improved. Furthermore, since L1 is equivalent to or larger than L2, an increase in a sliding resistance can be suppressed to a bare minimum. Therefore, an inserting posture of the piston can be stabilized without increasing a sliding resistance, thereby preventing the piston from wobbling.

For the syringe piston in accordance with the present invention, a minute circular groove and/or a minute circular protrusion can be formed on the sliding surface on the leading end side of the piston.

By the above configuration, a syringe piston having a more excellent sealing performance can be provided.

### EFFECT OF THE INVENTION

By the present invention, it is possible to provide a syringe piston having a high sealing performance caused by a stable inserting posture of the piston and having a sliding resistance that does not increase even in the case in which a force for disturbing an inserting posture of the piston is applied to the piston in a filling or use of the piston.

### BRIEF DESCRIPTION OF THE DRAWINGS

Fig. 1 is a cross-sectional view and a side elevational view for illustrating a syringe in which a syringe piston in accordance with an embodiment of the present invention is used.
Fig. 2 is a partially enlarged view expanding and showing a part of Fig. 1.
Fig. 3 is a partially enlarged view expanding and showing another part of Fig. 1.
Fig. 4 is a schematic view showing a state in which a syringe piston is inserted into a syringe barrel for the syringe of Fig. 1.

### EXPLANATIONS OF LETTERS OR NUMERALS

2: Piston
4: Plunger rod
5: Circular groove
6: Syringe barrel
8: Circular depression
R1: Diameter of a sliding surface on the leading end side of the piston
R2: Diameter of a sliding surface on the rear end side of the piston
B: Sliding surface on the leading end side
C: Sliding surface on the rear end side
D: Inner diameter of the syringe barrel
E: Flat part
r: Radius of curvature of a corner portion on the leading end side of the piston
L1: Length (area) in the axial direction of the sliding surface B
L2: Length (area) in the axial direction of the sliding surface C

### BEST MODE OF CARRYING OUT THE INVENTION

A preferable embodiment (example) of the present invention will be described below in detail.

A material that is used for producing a syringe piston for a medical drug and a medical treatment (hereafter simply referred to as a piston in some cases) in accordance with an embodiment of the present invention is not restricted in particular, and can be a rubber material, which has been used for producing a piston.

As a rubber material, there can be mentioned for instance a butyl series rubber such as a butyl rubber, a chlorinated butyl rubber, a brominated butyl rubber, and a divinylbenzene copolymerization butyl rubber; a conjugated diene series rubber such as a polyisoprene rubber, (high to low cis 1, 4 bonding), a polybutadiene rubber (high to low cis 1, 4 bonding), and a styrene-butadiene copolymerization rubber; and a thermoplastic elastomer such as an ethylene-propylene-diene terpolymerization rubber (EPDM), a styrene-ethylene-butadiene copolymer (SEBS), a block copolymer of styrene-butadiene-styrene (SBS), a styrene-isoprene block copolymer (SIS), and a styrene-isobutylene block copolymer (SIBS).

A thermoplastic elastomer is a material of a kind different from that of a rubber material in a precise sense. However, the thermoplastic elastomer is used similarly to a rubber material in the fields of the present invention. Consequently, a rubber material in accordance with the present invention includes a thermoplastic elastomer.

The piston in accordance with an embodiment of the present invention is produced by using a crosslinking rubber composition (compound) that is obtained by kneading the above rubber material and a compounding agent such as a crosslinking agent, a filler and/or a reinforcing agent, a coloring agent, and an age resister as needed and by a publicly known molding method such as a compression molding and an injection molding for a piston. A compounding agent to be used is not restricted in particular, and can be a compounding agent that has been used in producing a rubber stopper or a piston for a pharmacological product or medical treatment tools.

The syringe piston in accordance with an embodiment of the present invention can display the effects thereof for a laminate piston that requires the severe design conditions to ensure a sealing performance. However, it comes near to stating the obvious that the configuration of the present invention can also be applied to a syringe piston in which only a face that comes into contact with a medicinal solution is laminated (a partially laminate piston) and a syringe piston that is not laminated (a non laminate piston).

As a kind of a laminate film, there can be mentioned for instance a fluorine series resin, polypropylene, polyethylene, and ultrahigh molecular weight polyethylene. However, a kind of a laminate film is not restricted to the above materials.

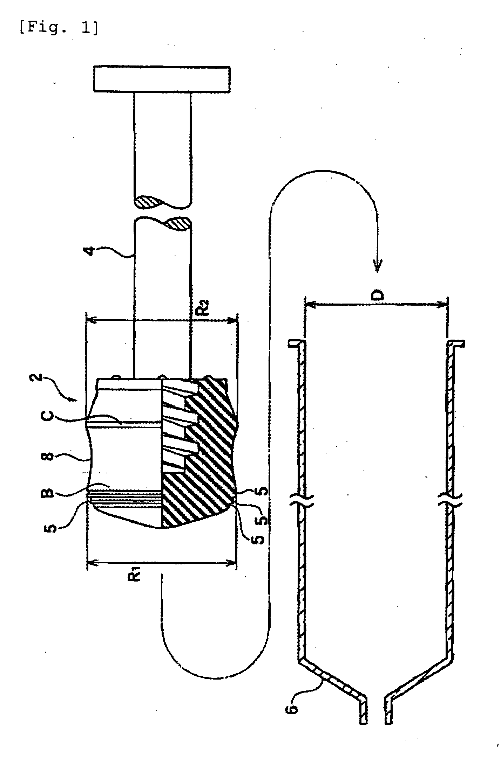

Fig. 1 shows a syringe in which a syringe piston for a medical drug and a medical treatment in accordance with an embodiment of the present invention is used.

A syringe piston 2 is in a generally cylindrical shape in which the axis line of a syringe barrel 6 is a center line of the syringe piston. The material of the syringe piston 2 is a rubber, and the production method of the syringe piston 2 is similar to the case of a conventional piston. However, a diameter of a sliding surface C on the rear end side that is connected to a plunger rod (a piston push rod) 4 by a screwing method is larger than that of a sliding surface B on the leading end side that comes into contact with a medicinal solution.

A minute circular groove 5 is formed on the sliding surface B on the leading end side that comes into contact with a medicinal solution. Moreover, a corner portion of the sliding surface on the leading end side that comes into contact with an inner wall of the syringe barrel 6 is formed in a circular arc shape in which a radius r of curvature is equivalent to or larger than 0.7 mm.

As described in detail below, a diameter of the sliding surface B and a diameter of the sliding surface C for the piston 2 are larger than an inner diameter D of the syringe barrel 6. Consequently, the piston 2 is compressed in the syringe barrel 6 when being mounted, thereby ensuring a sealing performance. A circular depression 8 is formed at an almost intermediate part of the piston 2 formed in a generally cylindrical shape. The sliding surface B and the sliding surface C are disposed on the both sides of the circular depression 8. In the case in which the maximum diameter of the sliding surface B on the leading end side of the piston is R1 and the maximum diameter of the sliding surface C on the rear end side of the piston is R2, R1 is smaller than R2.

The sliding surface B and the sliding surface C come into contact with the inner wall of the syringe barrel 6 in the case in which the piston 2 is inserted into the syringe barrel 6. However, the circular depression 8 does not come into contact with the inner wall of the syringe barrel 6.

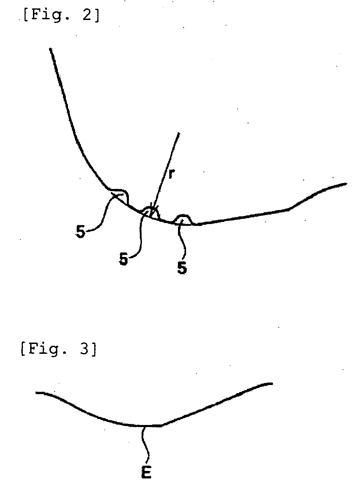

On the other hand, the corner portion of the sliding surface B on the leading end side and the sliding surface C on the rear end side of the piston 2 are formed in a circular arc shape. As shown in the partially enlarged view of Fig. 2, the circular arc formed on the corner portion on the leading end side of the sliding surface B is a circular arc having a radius r. The radius r is larger than a radius of a conventional piston.

In the case in which a radius r of curvature is less than 0.7 mm, a concentration of stress occurs to the corner portion. Consequently, a sliding resistance is increased, and a gap is generated between the piston and the inner wall of the syringe barrel even in the case in which the piston is disposed in a syringe barrel at a slight angle, thereby causing a solution leak to occur easily. On the other hand, in the case in which a radius r of curvature is larger than necessary, the piston becomes large and long, which is undesirable on a design of a syringe.

Consequently, a radius r of curvature of the corner portion on the leading end side of the piston is at least 0. 7 mm, preferably in the range of 0.9 to 2.0 mm, more preferably in the range of 0.9 to 1. 6 mm. This level of a radius r of curvature causes stress on the corner portion to be dispersed moderately and prevents the piston from being large and long. The optimum value of a radius r of curvature depends on a size of a piston. It is preferable that a radius r of curvature is in the range of 0.9 to 1.4 mm for a piston having a diameter of approximately 7 mm.

As shown in Figs. 1 and 2, a minute circular groove 5 is formed in the range of the circular arc having a radius r. Since the minute circular groove 5 is formed on the sliding surface B on the leading end side that comes into contact with a medicinal solution for the piston 2, a medicinal solution having a high penetration property is prevented from penetrating into the sliding surface B, thereby ensuring a high sealing performance. In addition, a sliding resistance is reduced advantageously. The reason is not known exactly, but it is thought that even in the case in which a medicinal solution starts to penetrate into the sliding surface B, the medicinal solution enters into the minute circular groove 5, whereby a penetration of the medicinal solution into the entire of the sliding surface B can be prevented.

A concentration of stress during a sliding of the piston occurs on the leading end portion having the maximum diameter of the piston (a medicinal solution contact side) in ordinary circumstances. It is thought that the reason why a sliding resistance is reduced by forming the minute circular groove 5 is that the concentration of stress is dispersed by a deformation of the minute circular groove 5.

Moreover, it is preferable that at least two minute circular grooves 5 are formed. A cross-sectional shape of the minute circular groove 5 is not restricted in particular, and can be any shape such as a semicircular shape and a triangular shape.

The groove is formed in the embodiment of the present invention. However, a protrusion (not shown) can also be formed for instance. Moreover, a combination of a groove and a protrusion (not shown) can also be implemented. Across-sectional shape of the protrusion is not restricted in particular similarly to the above described cross-sectional shape of the minute circular groove.

As shown in the partially enlarged view of Fig. 3, a flat part E is formed at the top part of a circular arc formed on the sliding surface C. Since the flat part E is formed on the sliding surface C, in the case in which the piston 2 is inserted into the syringe barrel 6, the flat part E comes into contact face-to-face with the inner circumference face of the syringe barrel 6. Consequently, a stability of an inserting posture of the piston 2 can be improved preferably.

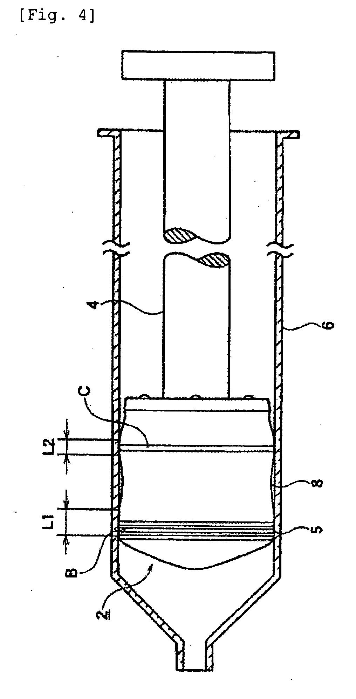

As shown in Fig. 4, in the state in which the piston 2 is inserted into the syringe barrel 6, in the case in which a length (an area) in the axial direction of the sliding surface B is L1 and a length (an area) in the axial direction of the sliding surface C is L2, the piston 2 is designed in such a manner that L1 is equivalent to or larger than L2. This is because a longer sliding surface B is advantageous for the purpose of holding a sealing performance to a medicinal solution. It is not required that the length L2 is larger for the purpose of controlling the posture of the piston 2. In addition, it is preferable that the length L2 is smaller for the purpose of reducing a sliding resistance.

As described above, for the piston 2 in accordance with the embodiment of the present invention, R1 is smaller than R2, L1 is equivalent to or larger than L2, r is equivalent to or larger than 0.7 mm, and a flat part E is formed.

For the piston 2 having the above configuration, in the case in which the piston 2 is inserted into the syringe barrel 6 or the piston 2 slides in the syringe barrel 6, an inserting posture of the piston 2 can be prevented from being disturbed. Even in the case in which an inserting posture of the piston 2 is slightly disturbed, a solution can be prevented from leaking. In addition, an increase in a sliding resistance can be suppressed.

While the preferred embodiments in accordance with the present invention have been described above, the present invention is not restricted to the embodiment.

The syringe piston in accordance with the embodiment of the present invention is suitable for a piston of a prefilled syringe that is filled with a medicinal solution having a high penetration property in advance. However, the present invention can also be applied to other types of syringe pistons. Moreover, a medicinal solution can be any of a one-component type and a two-component type.

## Claims

1. A syringe piston that is made of a rubber in a generally cylindrical shape and that is slidably mounted in a syringe barrel,
the syringe piston in a generally cylindrical shape comprising a circular depression at an almost intermediate part thereof,
wherein R1 is smaller than R2 in the case in which sliding surfaces are disposed on the both sides of the circular depression and R1 is a diameter of the sliding surface on the leading end side of the piston and R2 is a diameter of the sliding surface on the rear end side of the piston,
L1 is equivalent to or larger than L2 in the case in which L1 is a length of the sliding surface on the leading end side of the piston and L2 is a length of the sliding surface on the rear end side of the piston, and
r is equivalent to or larger than 0. 7 mm in the case in which a radius of curvature of a corner portion on the leading end side of the piston is r.

2. The syringe piston as defined in claim 1, wherein a flat part is formed on the sliding surface on the rear end side of the piston.

3. The syringe piston as defined in claim 1, wherein a minute circular groove and/or a minute circular protrusion are formed on the sliding surface on the leading end side of the piston.
